# EUROPEAN PATENT APPLICATION

(11) **EP 2 842 528 A1**
(43) Date of publication of application: **04.03.2015**
(21) Application number: 14250097.4
(22) Date of filing: 08.08.2014
(51) Int. Cl.: A61F 7/10, A61F 7/00, A61F 7/02

(54) **Apparatus and method for cooling head injury**

(30) Priority: 03.09.2013 TW 102216513
(71) Applicant: Ampac Enterprises Inc., Shirley, Massachusetts (US)
(72) Inventor: Chen, Ya-Chi, 244 New Taipei City (TW); Stanley Jr., Jurga, Shirley, Massachusett (US); Farrago, Douglas, Forest, VA 24551 (US); Rodrick, Austin, Cuyahoga Falls, Ohio 44223 (US)
(74) Representative: Whitaker, Iain Mark

(57) **Abstract**

An apparatus and method for treating a head injury including head gear and at least one cooling medium. The cooling medium has a prolonged cooling effect to aid injured persons recovery and health after sustaining a head injury. The apparatus and method is also contemplated for use with headaches, migraines, heat and/or sun stroke, as well as persons that have suffered a stroke.

## Description

### FIELD OF THE INVENTION

This invention relates to an apparatus and method for treating a head injury, brain injury, headaches, migraine, and sun or heat stroke. In particular, the present invention is directed to a cooling device to aid injured persons recovery and health after sustaining a head and/or brain injury or presenting symptoms related to a headache, migraine, sun or heat stroke, or the like. The apparatus and method of the present invention is also contemplated for use with persons suffering from a stroke. The method of the present invention is contemplated for use at any point after head and/or brain injury or stroke, but earlier application of the apparatus to the injured person is preferred in such cases.

The apparatus and method of the present invention is also contemplated for non-injured persons exposed to head impact. In such cases, earlier and regular application of the apparatus to those non-injured persons exposed to head impact is preferred. Use of the method and apparatus of the present invention for persons engaging in activities that are likely to involve head impact is also contemplated.

### BACKGROUND OF THE INVENTION

Head injuries and other serious medical conditions are prevalent in certain sports. The American Academy of Neurology recently released a comprehensive study in the journal Neurology that confirms significant previously held theories that head injuries cause neurologic problems later in life. The study, which followed 3,439 retired professional football players, found that retired professional football players are three times more likely than the rest of the population to have neurodegenerative diseases and four times more likely to develop amyotrophic lateral sclerosis ("ALS"), also known as Lou Gehrig's disease. In addition, the study found a higher incidence of Alzheimer's and Parkinson's disease in the retired professional football players as compared to the general population. Finally, the study found that the players were at an increased risk of dying from a neurological disorder, as compared to the general population. The American Academy study found that the velocity players, the running backs, the wide receivers, and the punt and kickoff returners, were more likely to develop problems because they were more susceptible to violent hits than other position players.

Similarly, 15 to 40 percent of ex-boxers have been found to have symptoms of chronic brain injury. In fact, recent studies have shown that most professional boxers (even those without symptoms) have some degree of brain damage. Indeed, the force of a professional boxer's fist is equivalent to being hit with a 13-pound bowling ball traveling 20 miles per hour, or about 52 times the force of gravity. Likewise, a McGill University study found that more than 60 percent of college-level soccer players reported symptoms of concussion during a single season. Cheerleading, cycling, horseback riding, and snow skiing and snowboarding are examples of other sports that pose risk of traumatic brain injury.

Even of the sports that require protective headgear during play, helmets and other such headgear offer no protection against a concussive brain injury because concussions occur when a fast-moving body suddenly stops or changes direction. In other words, the brain keeps moving until it collides with the inside of the skull, which causes damage that can lead to chronic traumatic encephalopathy ("CTE"). Indeed, studies have repeatedly linked concussions in football players to chronic traumatic encephalopathy (CTE).

Leading research points to rotational accelerations as a leading cause of concussions (as opposed to linear or straight ahead impacts). Without being bound to any particular theory, it is believed that the rotational accelerations force the skull to rotate around the brain because the inertia of the brain keeps it stationary. As a result, a large amount of connective tissue around the circumference of the brain is sheared. Even if worn, helmets and other such headgear cannot necessarily prevent rotational acceleration.

For example, when a helmeted football player charging down the field is blocked by a hit to the chest, his body will stop but the head will rotate forward as in a whiplash motion. Similarly, even if a boxer wears a sparring helmet, a punch to the side of the head will force the head to spin in a rotational direction. Without being bound to any particular theory, it is believed that there are some cases where the use of the sparring helmet may result in a more damaging rotational acceleration because the helmet may allow for higher leverage / torque on the head. Furthermore, there exists a theory that a high percentage of soccer players suffer concussions because of the head rotation that occurs when "heading" a soccer ball. In particular, the violent snap of the neck and rotation of the head (and perhaps the sudden stopping of the head) when contacting the ball are rotational accelerations that shear a large amount of brain tissue.

Accordingly, there is a growing anxiety about head injuries in these sports. And, while more is known about concussions today and the link to long-term neurologic problems, there is little known about methods to prevent head injuries, manage head injuries, and treat head injuries other than rest. In fact, normal care for a severe brain injury is sedation and ventilation.

Recent research discusses hypothermia as a potent neuroprotectant. For example, "The Use of Hypothermia as a Treatment for Traumatic Brain Injury" authored by Kristin Rupich generally provides that hypothermia protects the brain by inhibiting massive depolarization in the brain and the release of glutamate and asparate. As such, hypothermia stabilizes the blood-brain barrier and prevents cell death. Hypothermia may also prevent uncoupling of the metabolic supply demand regulation and prevent loss of cerebral autoregulation. However, there is not enough research available to define the parameters for a method of cooling, a goal temperature, or duration of cooling and the use of hypothermia for head injuries is still considered under development.

For example, recent experimentative treatment for brain swelling after severe head trauma, which includes a cooling treatment via an intravenous drip and frozen pads put on the person's back and chest for at least about 48 hours to reduce body temperature to about 89.6°F to 95°F from the normal 98.6°F, has promising results. In addition, studies have already shown that babies who are starved of oxygen at birth are much less likely to suffer brain damage if they receive cooling treatment. In particular, a thin plastic cap with cold water (between about 50°F and 59°F) circulating inside is placed on the infant's head in an effort to cool the infant's body temperature to 94°F.

In addition, emergency services professionals / first responders are now being encouraged to treat stroke victims immediately after diagnosis by cooling the neck and head in an attempt to reduce cytotoxic cascade, stabilize the blood-brain barrier, reduce free radical formation, and prevent neurotoxicity of tPA.

As such, there remains a need in the art to aid recovery when head injuries occur. Similarly, there remains a need in the art for an apparatus and method to alleviate the symptoms associated with headaches, migraines, sun or heat stroke, and other similar conditions. It would also be beneficial to use such an apparatus for use in selective hypothermia with stroke victims.

### SUMMARY OF THE INVENTION

The present invention is directed to a device for thermally regulating the brain comprising: head gear capable of being placed on the head of an individual; and at least one cooling medium, wherein the at least one cooling medium has a first temperature and a second temperature after about 1 hour of use that is greater than the first temperature.

In one embodiment, the first temperature is about 8°F to about 12°F. In another embodiment, the second temperature is about 35°F to about 65°F. For example, the second temperature may range from about 35°F to about 45°F.

The present invention is also directed to an article of headwear having at least one cooling medium removably supported therein, comprising: head gear comprising at least a crown section; at least one pouch secured inside the crown section; and at least one cooling medium shaped to be received and stored in at least one pouch, wherein the at least one cooling medium has a first temperature and a second temperature after about 90 minutes of use that is greater than the first temperature.

In one embodiment, the headwear further comprises a neck portion. In another embodiment, the first temperature is about 5°F to about 15°F. In still another embodiment, the second temperature is about 32°F to about 65°F. For example, the second temperature may about 35°F to about 45°F.

The present invention is also directed to a method for treating brain trauma comprising: providing head gear comprising at least a crown section, at least one pouch secured inside the crown section, a neck portion optionally comprising at least one pouch secured therein, and at least one cooling medium shaped to be received and stored in the at least one pouch secured inside the crown section, the at least one pouch secured in the neck portion, or a combination thereof, wherein the at least one cooling medium has a first temperature and a second temperature after a predetermined period of time that is greater than the first temperature; securing the head gear onto a person for the predetermined period of time.

In one embodiment, the second temperature is about 32°F to about 65°F. In another embodiment, the second temperature is about 35°F to about 45°F. In still another embodiment, the first temperature is about 2°F to about 15°F. For example, the first temperature may be from about 8°F to about 12°F. In yet another embodiment, the predetermined period of time is about 30 minutes to about 2 hours. For example, the predetermined period of time may be from about 30 minutes to about 90 minutes.

### BRIEF DESCRIPTION OF THE DRAWINGS

Further features and advantages of the invention can be ascertained from the following detailed description that is provided in connection with the drawings described below:
FIGS. 1A and 1B illustrate a side view of the device according to one embodiment of the invention;
FIGS. 2A and 2B illustrate a perspective view of the device according to another embodiment of the invention;
FIGS. 3A-3F illustrate side, front, and rear views of the device according to yet another embodiment of the invention;
FIGS. 4A-4B show a front view of the inside of the device according to one embodiment of the invention;
FIGS. 5A-5B show an embodiment of the cooling medium according the present invention; and
FIG. 6 shows a cooling medium according to one embodiment of the invention.

### DETAILED DESCRIPTION OF THE INVENTION

As mentioned above, the present invention is directed to a cooling device intended to aid injured person's recovery and health after sustaining a head injury. In addition, the apparatus and method of the present invention is contemplated for use in persons suffering from a headache, migraine, sun or heat stroke, or the like, as well as persons suffering from a stroke.

Without being bound by any particular theory, it is now believed that cooling the brain after a concussive head injury and/or stroke aids both in short-term recovery and may help to reduce long-term effects. In particular, it is believed that brain cooling helps reduce swelling and pressure in the skull after severe head injury. For example, the primary injury, *i.e.,* the damage that occurs at the time of impact, is different from the biochemical and cellular response to the primary injury that begins at within minutes to hours of the primary injury ("secondary injury"). The secondary injury is global and is believed to harm tissue that was not involved in the primary injury. In this regard, the inventors contemplate that cooling or hypothermia treatment of the head and/or neck immediately after impact will minimize or prevent the secondary injury and also aid in addressing the damage from the primary injury (*e.g.,* by facilitating restoration of membrane function, attenuating cytoskeletal damage, limiting axonal damage, and reducing apoptosis).

Accordingly, a device and method that cools the brain after a concussive head injury are contemplated in the present invention. Similarly, the device and method discussed herein are also contemplated for use with headaches, migraines, sun or heat stroke, and the like, as well as post-stroke. Likewise, the device and method described herein are contemplated for use with the non-injured player or person for preventative case. The device and method of use are discussed in greater detail below.

### The Device

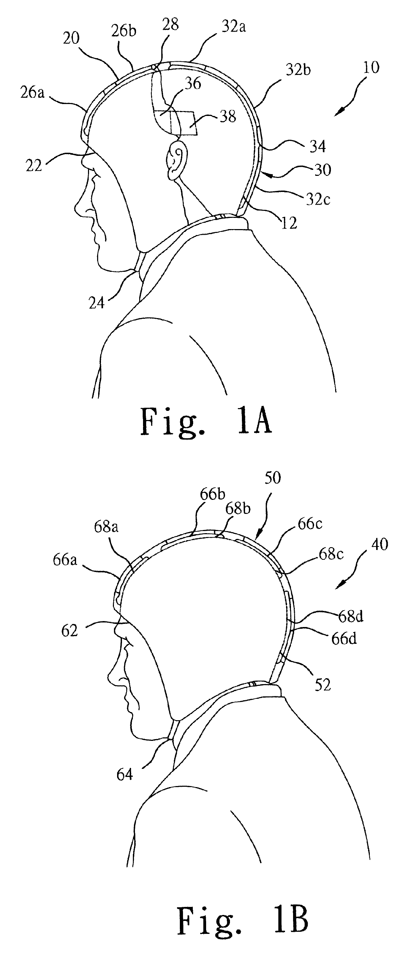

The device includes a head gear capable of receiving at least one cooling medium and at least one cooling medium capable of being secured to the head gear. The head gear may take a variety of forms providing it accommodates at least one cooling medium attached or removably coupled thereto. For example, in one embodiment, the device 10 includes a plurality of head gear components, each head gear component being equipped with at least one coupling area to which the cooling medium may be attached or removably coupled thereto. As shown in FIG. 1A, the head gear may include a front component 20 and a rear component 30 that are fit over / onto the head 12. The front and rear components may be formed such that the ears are exposed. Similarly, the front component 20 may be formed with a cut out 22 for the face and a chin strap to secure the head gear 10 onto the head 12. The front and rear components may also include at least one tab (28, 36) to secure the components to each other over the head 12.

The front and rear components may also each include at least one coupling area (26, 32) to which the cooling medium may be attached or removably coupled thereto. For example, in one embodiment, the cooling medium(s) may be removed from the head gear front and rear components 20, 30 for storage, cleaning / sterilizing and cooling purposes. Similarly, the cooling medium(s) may be disposable in nature and thus intended to be removed and disposed of after a single use. Such disposable / single use cooling medium(s) are discussed in greater detail below. In another embodiment, the cooling medium(s) is permanently attached such that the entire head gear front component 20 and/or rear component 30 are cooled before use.

As shown in FIG. 1A, a plurality of coupling areas (26a, 26b) may be used to secure one cooling medium 28 to the front head gear component 20. In the alternative, each coupling area may be used for a separate cooling medium 28. Similarly, a plurality of coupling areas (32a, 32b, and 32c) may be used to secure one cooling medium 34 to the rear head gear component 30. In the alternative, each coupling area may be used for a separate cooling medium 34.

In another embodiment, the device 40 may include a unitary piece for the head gear 50, as shown in FIG. 1B. In particular, the head gear 50 fits over the head 52. The head gear component may have an opening for the face 62, a chin strap 64, and at least one coupling area 66 on the inside of the head gear 50 for attaching or removably coupling the cooling medium thereto. As shown in FIG. 1B, multiple coupling areas (66a-66d) may be used in the head gear to which at least one cooling medium is attached or removably coupled thereto. In the alternative, multiple coupling areas may be used to secure one cooling medium.

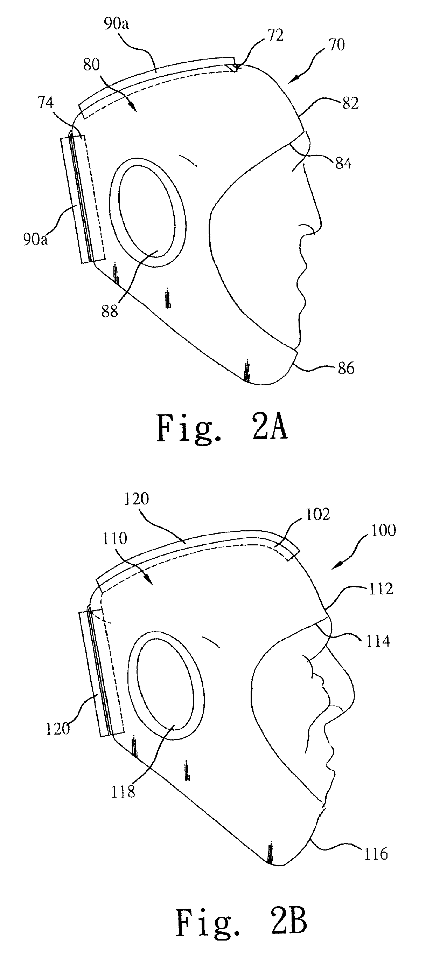

As shown in FIGS. 2A and 2B, the device 70, 100 may use a head gear 80, 110 that takes the form of a head mask with ear openings 88, 118. The head gear 80, 110 has an upper part 82, 112, a cut out for the face 84, 114, and a lower part 86, 116. In FIG. 2A, the crown 72 and rear 74 of the head gear 80 are open to allow the cooling medium (90a, 90b) to be secured to these portions of the head gear and changed out during the cooling treatment if necessary without removing the head gear 70. In another aspect of the invention, as shown in FIG. 2B, a contiguous portion 102 of the head gear 110 that encompasses the crown and rear of the head gear is open to receive the cooling medium 120.

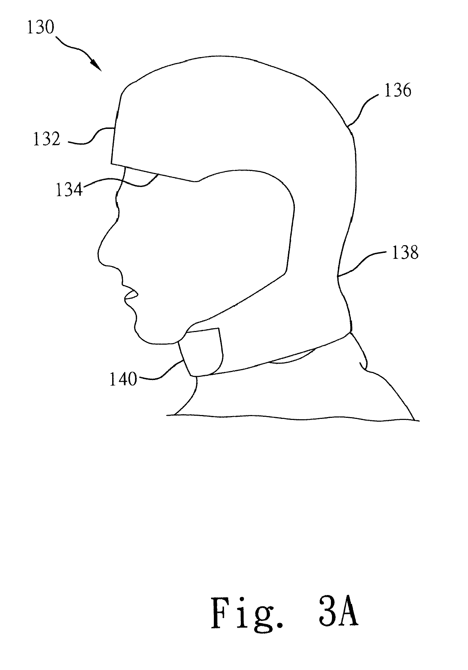

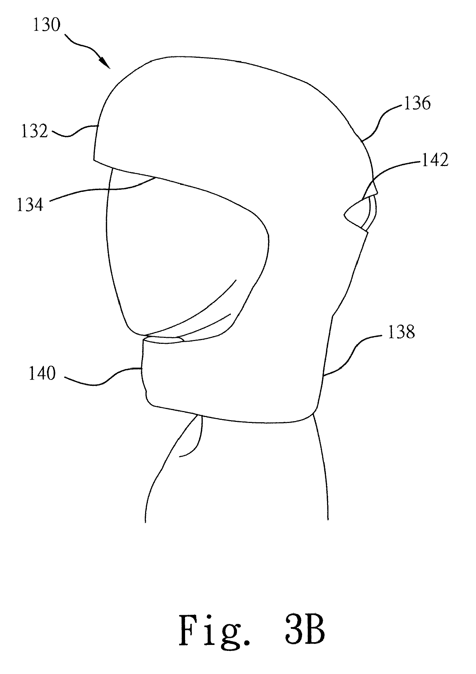

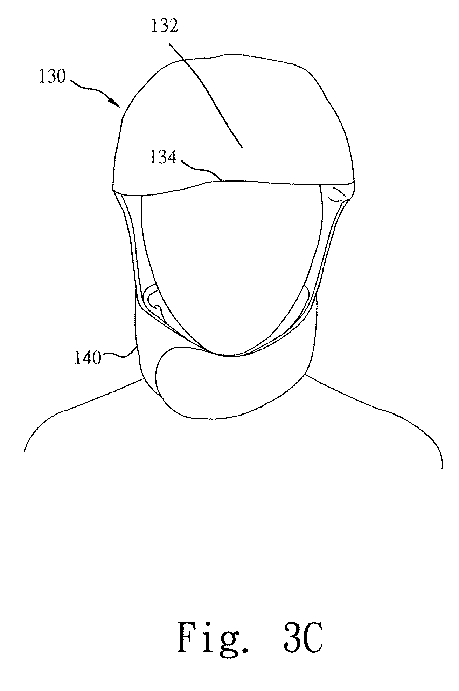

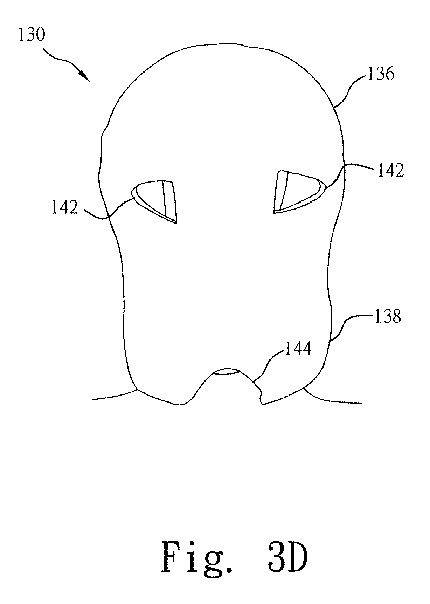

FIGS. 3A-3D shows an embodiment of the invention where the head gear 130 includes an upper portion 132 that at least partially covers / contacts the forehead, a cut out for the face 134, a rear portion 136, and a neck portion 138 that wraps around the neck via closure 140 (as shown) or covers at least a portion of the back of the neck (not shown). The rear portion 136 may include a plurality of openings 142, as shown in FIG. 3D, as well as a notch 144 in the rear neck area for comfort. In another embodiment, the neck portion is not integrated with the head gear. Rather, the neck portion is a separate neck collar.

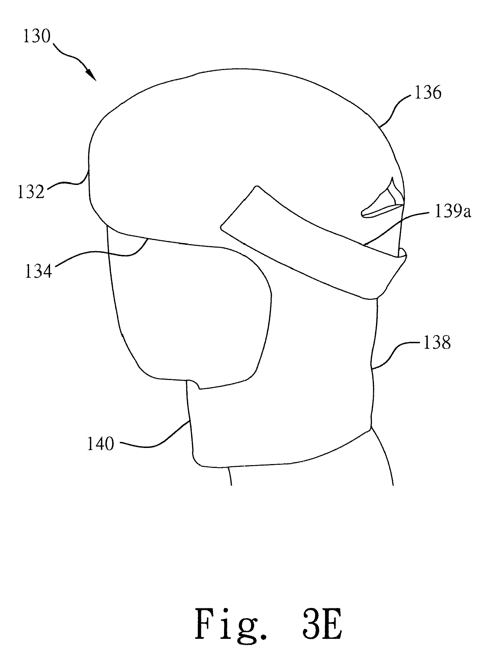

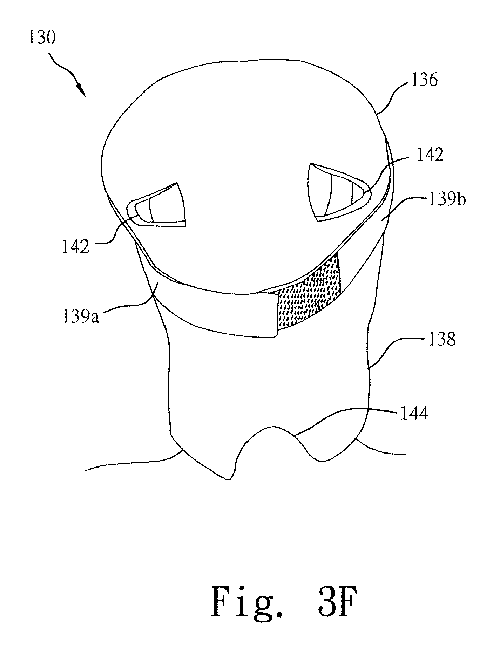

As shown in FIGS. 3E and F, the head gear 130 may be equipped with a securing system. In one embodiment, the securing system includes at least two straps that may be removably coupled at the rear of the headgear 130. For example, as shown in FIG. 3E, strap 139a may be attached to a first side portion of the head gear 130. Strap 139b (not shown in FIG. 3E) may be attached to a second side portion (opposite to the first side portion) of the head gear 130. As shown in FIG. 3F, the straps 139a and 139b may be removably coupled at the rear of the headgear 130 to tighten / secure the headgear 130 to various head sizes during use for better thermal conductivity and/or a more secure fit. In one embodiment, at least one of straps 139a and 139b includes hooks and the other strap includes loops to affect a hook-and-loop coupling once pressed together. In another embodiment, the straps 139a and 139b may be removably coupled using buttons, snaps, or other similar coupling devices that may be removably attached. In one embodiment, the straps 139a and 139b may be joined near or just below the occipital lobe. The securing system may be formed from an elastic material.

While only two straps are shown in FIGS. 3E and 3F, less than or more two straps are contemplated. For example, in one embodiment, the head gear may include multiple straps. Similarly, while the figures show the removably coupled nature of the straps, a fastening system for the head gear may include a unitary strap or straps that traverse from side to side of the head gear. In such an embodiment, the straps may be formed from an elastic material such that the strap(s) may be pulled over and rest below the occipital lobe.

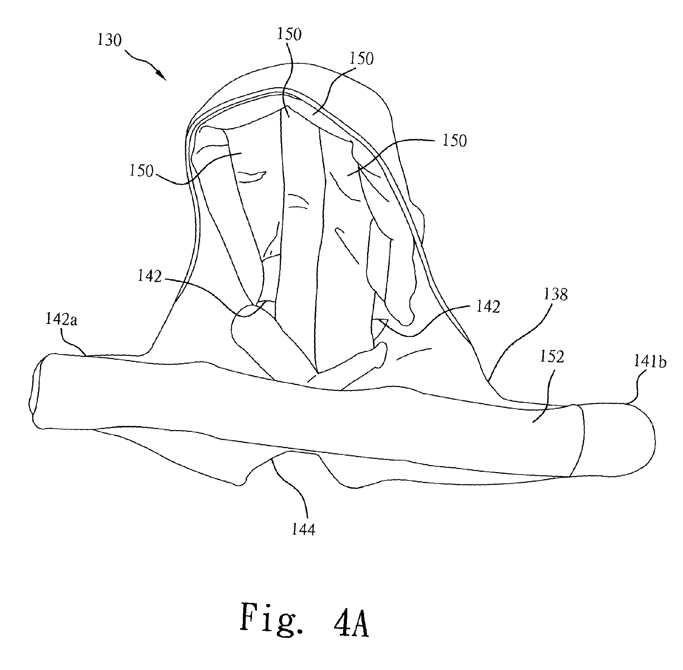

As shown in FIG. 4A, the neck portion 138 of head gear 130 includes tabs 141a and 141b that are removably coupled to secure the head gear 130 about the front of the neck. The tabs 141a and 141b may be removably coupled using a hook-and-loop fastener system, buttons, snaps, or other similar coupling devices that allow for ease of removal.

The figures are not intended to limit the number of coupling areas, cooling mediums, or head gear components used in the device. For example, the cooling means may be placed into one or more pouches in the head gear. In one embodiment, the pouches include two layers of different materials. In particular, the first layer or outer layer is formed of a thin material having good thermal transmissive and waterproofing properties. The second layer or inner layer (the layer that will contact the cooling medium) may have thermal insulative properties. Such a pouch design has an overall effect of permitting heat to be absorbed by the cooling medium from the head of the injured person without at the same time absorbing excessive heat from the environment. In one embodiment, the material used to form the first layer include materials such as those described in U.S. Patent Nos. 5,283,112 and 5,239,037, the entire disclosures of which are incorporated by reference herein. The material used to form the second layer may include materials, such as those disclosed in U.S. Patent Nos. 5,295,267, 5,236,770, 5,230,922, 5,214,804, the entire disclosures of which are incorporated by reference herein.

The head gear 10 and 50 (exclusive of the coupling areas and tabs) may be formed from a flexible material. Suitable materials for use in the head gear 10 and 50 include, but are not limited to, natural and synthetic rubbers such as neoprene, synthetic resins such as silicone, synthetic polymers such as polyamides (e.g., nylon), flexible foam such as foamed polyurethane, unfoamed polyurethane, plastic, thermal fabrics such as polyester and micro fiber, and combinations thereof. In one embodiment, the head gear is formed from foamed or unfoamed neoprene. In another embodiment, the head gear is formed from a non-toxic synthetic rubber commercially available from Ariaprene™. In still another embodiment, the head gear is formed from silicone.

In yet another embodiment, the head gear is formed from a laminate material. For example, in this aspect of the invention, the head gear includes a plurality of layers that are laminated or bonded together to produce the laminate material. In particular, the head gear 10 may include two outer layers of a first material with high elasticity, e.g., nylon, and an inner layer disposed between the two outer layers of a second material that also has high elasticity, e.g., polyurethane. The laminated nature of the material, further examples of which are described in U.S. Patent No. 5,735,807, the entire disclosure of which is incorporated by reference herein, allows for a thin head gear with high strength and high elasticity.

In another embodiment, the head gear includes a plurality of segments that are linked together. In this aspect, the segmented portions may be formed from rigid materials that are linked together.

As shown in FIGS. 4A-4B, the inside of the head gear may include hook and loop fastener compatibility such that the cooling medium may be secured in place when in use, but may be removed before or after use. Other suitable methods to secure the cooling medium are contemplated such as snaps, buttons, and the like. For example, one or more cooling mediums 150 may be removably secured in the head area of the head gear 130. Similarly, one or more cooling mediums 152 may be removably secured in the neck area of the head gear 130,

According to one embodiment of the present invention, the cooling device is integrated within a garment. For example, the cooling device may be integrated into the hood of a sweatshirt such that the injured person can stay relatively warm around the torso while receiving the cooling treatment around the head.

In one embodiment, the head gear excludes any metallic components so as to enable use of the cooling device while receiving a magnetic resonance image (MRI).

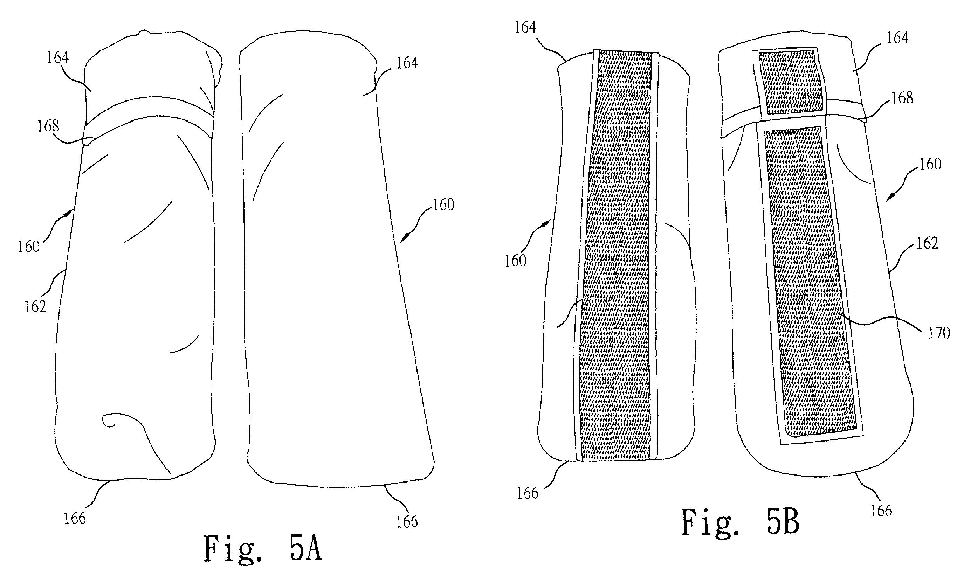

The cooling medium may take a variety of forms. For example, as shown in FIG. 5A, the cooling medium may include at least one external sleeve 160 that includes a tube 162 having a top end 164 and a bottom end 166. The bottom end 166 may be sewed or otherwise closed. The top end 164 may include a flap / opening 168 to allow placement of the cooling medium. The removal of the cooling medium from the external sleeve allows for easier cleaning and disinfecting and also allows the cooling medium to be frozen without the entire device.

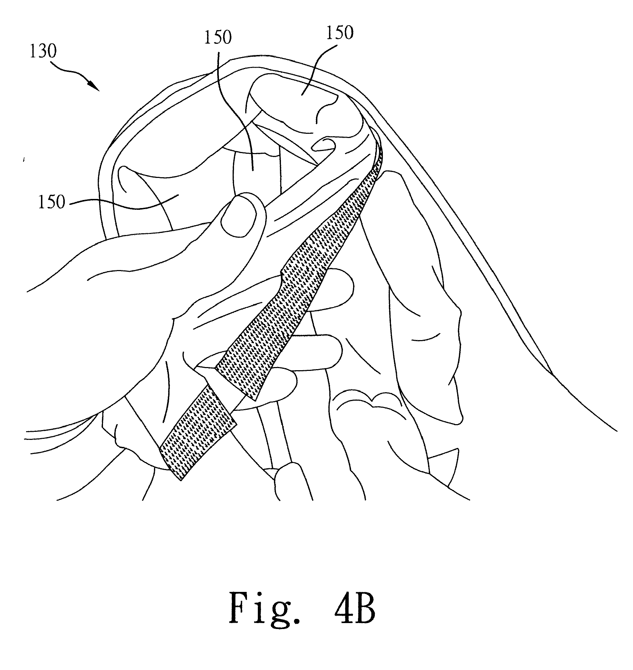

One side of the sleeve 160 may include hook and loop fastener compatibility to allow for removable placement as shown in FIG. 4B. For example, as shown in FIG. 5B, the sleeve 160 may include at least one portion 170 including hooks or loops such that the mating portion within the inside of the head gear 130 may removably attach.

In one embodiment, the cooling medium includes a single sleeve capable of containing a fluid. The fluid may be a refrigerant gel or liquid that is capable of absorbing a considerable amount of heat. In other words, the fluid preferably has a high enthalpy of fusion. In another embodiment, the fluid may be a solution that includes a first liquid and at least one additive.

Non-limiting examples of the first liquid include propylene glycol, ethylene glycol, short chains alcohols such as ethyl alcohol and methyl alcohol, isopropyl alcohol, and glycerol, and combinations thereof. Typical refrigerants such as ammonia, carbon dioxide, and non-halogenated hydrocarbons may also be used as a first liquid. Deicers and antifreezes are also contemplated for use as the first liquid. For example, calcium chloride may be used as the first liquid. Suitable additives may include, but are not limited to, substances to prevent bacterial growth, substances that cause the first liquid to remain a gel throughout use, substances to depress the melting point, and combinations thereof. In one embodiment, the additive may include hydroxyethyl cellulose, vinyl-coated silica gel, or a combination thereof. In another embodiment, the additive is saline.

Aqueous solutions are also contemplated with the understanding that the amount of water in the solution will affect the freezing point. For example, a propylene glycol-water mixture with at least about 30 percent propylene glycol will have a freezing point of about 10°F whereas a propylene glycol-water mixture with at least about 60 percent propylene glycol will have a freezing point of about -60°F. In addition, any of the first liquids may be used in a blend with a suitable second first liquid. In one embodiment, the first liquid is a blend of propylene glycol and ethylene glycol. In still another embodiment, the first liquid is gelatin.

In another embodiment, the cooling medium includes a primary hollow and pliable container housing a first liquid and at least one secondary hollow container housing a second liquid. The first liquid has a first freezing point and the second liquid has a second freezing point greater than first freezing point. In one embodiment, the first liquid has a freezing point that ranges from about 10°F to about -90°F. In another embodiment, the first liquid has a freezing point that ranges from about 0°F to about -85°F. In still another embodiment, the freezing point of the first liquid is about -5°F to about -80°F. For example, the first liquid may have a freezing point of about -10°F to about -75°F. The aforementioned first liquids and aqueous solutions may be used in this embodiment with or without the additives.

The second liquid may have a freezing point of greater than about 5°F. In one embodiment, the freezing point of the second liquid is about 10°F or greater. In another embodiment, the freezing point of the second liquid is about 20°F or greater. In still another embodiment, the freezing point of the second liquid is about 30°F or greater.

Suitable second liquids include, but are not limited to, water, phenol, nitrobenzene, naphthalene, iodine, benzene, formic acid, acetic acid, saline, and ethylene bromide.

In yet another embodiment, the cooling medium includes bentonite clay (aluminum phyllosilicate).

Instant cooling packs are also contemplated for use as the cooling medium with the present invention. In other words, cooling packs that contain a fluid or gel that, upon activation, undergo an endothermic reaction are contemplated for use with the present invention. In one embodiment, activation may occur by squeezing, crushing, or otherwise manipulating the cooling pack to produce the endothermic reaction. Further examples of such cooling packs are described in U.S. Patent Nos. 6,497,116, 6,908,956, and 7,055,575, and U.S. Patent Publication No. 2005/0145372, the entire disclosures of which are incorporated by reference herein.

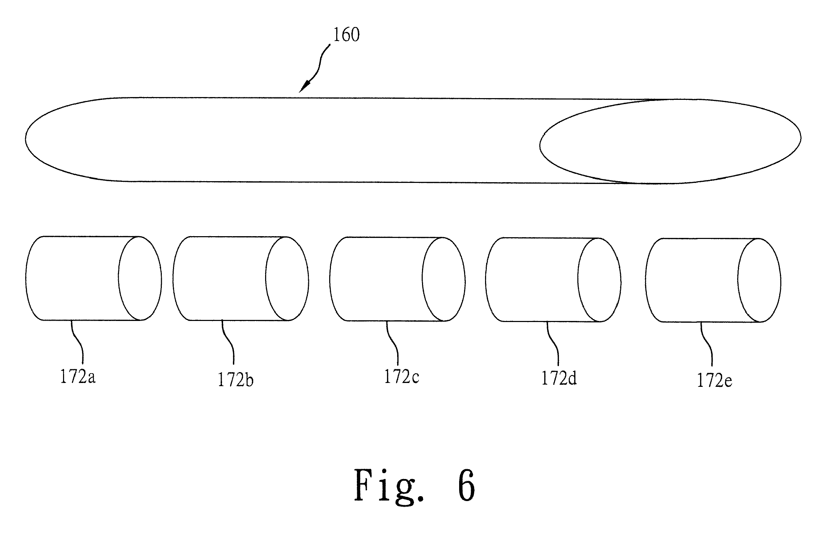

In one embodiment, the cooling medium is disposable. For example, it is also contemplated that the use of ice and/or water in a bladder may be used inside of a sleeve. The bladder may include any type of closure, *e.g.,* a screw-on lid. In another embodiment, the cooling mediums are fluid-filled sacks where the fluid is disposable. The sacks may be segmented for comfort, as shown in FIG. 6. For example, a sleeve 160 may include a plurality of fluid-filled sacks 172a-e. Any number of sacks may be used to allow for a malleable formation to the head of the user within the head gear.

The device of the present invention is preferably used with an initial temperature at the surface ranging from about 2°F to about 15°F. In one embodiment, the device has an initial surface temperature of about 5°F to about 12°F upon initiation of use. In another embodiment, the device has an initial surface temperature of about 8°F to about 10°F upon initiation of use.

The temperature at which the device may be used, *i.e.,* the application temperature, is preferably about 32°F or greater. In one embodiment, the time required for the device to reach the application temperature from the initial temperature may range from about 2 seconds to about 5 minutes. In one embodiment, the time required for the device to reach the application temperature from the initial temperature may range from about 10 seconds to about 3 minutes.

In another embodiment, the cooling device of the present invention remains cool at a temperature ranging from about 32°F to about 65°F for at least about 1 hour. In yet another embodiment, the cooling device of the present invention remains cool at a temperature ranging from about 35°F to about 55°F for at least about 1 hour. In still another embodiment, the cooling device of the present invention remains cool at a temperature ranging from about 35°F to about 45°F for at least about 1 hour. For example, the temperature of the cooling device after about 1 hour of use may range from about 32°F to about 45°F, about 41 °F to about 44°F, about 42°F to about 46°F, or ranges therebetween. In one embodiment, the temperature of the cooling device is no more than about 200 percent of the application temperature after about 1 hour. In another embodiment, the cooling device temperature is no more than about 150 percent of the application temperature after about 1 hour. In yet another embodiment, the cooling device temperature is no more than about 125 percent of the application temperature after about 1 hour. In still another embodiment, the cooling device temperature is no more than about 105 percent of the application temperature after about 1 hour.

In one embodiment, the temperature of the cooling device after about 75 minutes of use ranges from about 32°F to about 65°F. For example, the temperature of the cooling device after about 75 minutes of use may range from about 35°F to about 60°F, about 40°F to about 55°F, about 45°F to about 50°F, 32°F to about 45°F, or ranges therebetween. In another embodiment, the temperature of the cooling device after about 90 minutes of use ranges from about 32°F to about 70°F. For example, the temperature of the cooling device after about 90 minutes of use may range from about 40°F to about 70°F, about 45°F to about 70°F, about 55°F to about 70°F, 32°F to about 45°F, or ranges therebetween.

In another embodiment, the temperature of the cooling device after about 2 hours of use ranges from about 32°F to about 75°F. For example, the temperature of the cooling device after about 2 hours of use may range from about 40°F to about 75°F, about 45°F to about 75°F, about 55°F to about 75°F, 32°F to about 45°F, or ranges therebetween. In still another embodiment, the temperature of the cooling device after about 3 hours of use ranges from about 32°F to about 80°F. For example, the temperature of the cooling device after about 3 hours of use may range from about 40°F to about 80°F, about 45°F to about 80°F, about 55°F to about 80°F, 32°F to about 45°F, or ranges therebetween. In yet another embodiment, the temperature of the cooling device after about 4 hours of use may range from about 32°F to about 80°F, about 45°F to about 80°F, about 55°F to about 80°F, 32°F to about 50°F, or ranges therebetween.

For example, the cooling device may have an initial surface temperature of about 10°F upon removal from the freezer, about 32°F to about 40°F after about one hour of use, and about 40°F to about 50°F after about two hours of use.

### Method of Use

If the cooling medium is stored separately from the head gear, the cooling medium may be placed into / onto the device before / after the head gear is placed on the injured person's head. For example, in the embodiments shown in FIGS. 1A-1B and 3A-3D, the cooling medium is placed inside the head gear and then mounted on the head of the injured person. In the embodiments shown in FIGS. 2A and 2B, the head gear is mounted on the head of the injured person and then the cooling medium may be placed onto the device.

The head gear may be worn by the user for a predetermined period of time. In one embodiment, the predetermined period of time is about 30 minutes to about 2 hours. In another embodiment, the predetermined period of time is about 30 minutes to about 90 minutes. In still another embodiment, the predetermined period of time is about 30 minutes to about 75 minutes. For example, the user may apply the head gear for about 45 minutes.

In one embodiment, the head gear is contemplated for use during movement. In particular, the securing system shown in FIGS. 3E and 3F may enable a user to tighten the fit and remain mobile during use.

Although the present invention has been described with reference to particular embodiments, it will be understood to those skilled in the art that the invention is capable of a variety of alternative embodiments within the spirit of the appended claims. For example, the device and method of the invention are also contemplated for use as a preventative treatment to attenuate the accumulative effect of linear and rotational accelerations to the brain. In addition, the device and method of the invention is contemplated for use in enhancing on-field athletic performance, e.g., through use of the device before, during, and after exercise.

## Claims

1. A device for thermally regulating the brain comprising:
head gear capable of being placed on the head of an individual; and
at least one cooling medium, wherein the at least one cooling medium has a first temperature and a second temperature after about 1 hour of use that is greater than the first temperature.

2. The device of claim 1, wherein the first temperature is about 8°F to about 12°F.

3. The device of claim 2, wherein the second temperature is about 35°F to about 65°F.

4. The device of claim 3, wherein the second temperature is about 35°F to about 45°F.

5. An article of headwear having at least one cooling medium removably supported therein, comprising:
head gear comprising at least a crown section;
at least one pouch secured inside the crown section; and
at least one cooling medium shaped to be received and stored in at least one pouch,
wherein the at least one cooling medium has a first temperature and a second temperature after about 90 minutes of use that is greater than the first temperature.

6. The article of headwear of claim 5, further comprising a neck portion.

7. The device of claim 5, wherein the first temperature is about 5°F to about 15°F.

8. The device of claim 5, wherein the second temperature is about 32°F to about 65°F.

9. The device of claim 5, wherein the second temperature is about 35°F to about 45°F.

10. A method for treating brain trauma comprising:
providing head gear comprising at least a crown section, at least one pouch secured inside the crown section, a neck portion optionally comprising at least one pouch secured therein, and at least one cooling medium shaped to be received and stored in the at least one pouch secured inside the crown section, the at least one pouch secured in the neck portion, or a combination thereof, wherein the at least one cooling medium has a first temperature and a second temperature after a predetermined period of time that is greater than the first temperature; and
securing the head gear onto a person for the predetermined period of time.

11. The method of claim 10, wherein the second temperature is about 32°F to about 65°F.

12. The method of claim 11, wherein the second temperature is about 35°F to about 45°F.

13. The method of claim 10, wherein the first temperature is about 2°F to about 15°F.

14. The method of claim 13, wherein the first temperature is about 8°F to about 12°F.

15. The method of claim 10, wherein the predetermined period of time is about 30 minutes to about 2 hours.

16. The method of claim 10, wherein the predetermined period of time is about 30 minutes to about 90 minutes.
